# EUROPEAN PATENT SPECIFICATION

(11) **EP 4 534 215 B1**
(45) Date of publication and mention of the grant of the patent: **09.09.2026**
(21) Application number: 24150326.7
(22) Date of filing: 04.01.2024
(51) Int. Cl.: B06B 1/06, A61B 8/00, H10N 30/01, H10N 30/50, H10N 30/80, H10N 30/88

(54) **ULTRASOUND PROBE, METHOD OF MANUFACTURING THE SAME, AND STRUCTURE COMBINABLE WITH MAIN BACKING LAYER OF THE SAME**
ULTRASCHALLSONDE, VERFAHREN ZU IHRER HERSTELLUNG UND MIT IHRER HAUPTRÜCKSEITE KOMBINIERBARE STRUKTUR
SONDE ULTRASONORE, SON PROCÉDÉ DE FABRICATION ET STRUCTURE POUVANT ÊTRE COMBINÉE AVEC UNE COUCHE DE SUPPORT PRINCIPALE DE CELLE-CI

(30) Priority: 04.10.2023 KR 20230132057
(43) Date of publication of application: 09.04.2025
(73) Proprietor: Samsung Medison Co., Ltd., Hongcheon-gun, Gangwon-do 25108 (KR)
(72) Inventor: KYUNG, Yoonsung, 16017 Gyeonggi-do (KR); KWON, Sungdo, 05340 Seoul (KR); SHIM, Dongwoo, 16555 Gyeonggi-do (KR)
(74) Representative: Frey, Sven Holger

(56) References cited:
- EP-A1- 2 025 414
- US-A1- 2016 066 885
- US-A1- 2018 062 069

## Description

### CROSS-REFERENCE TO RELATED APPLICATION(S)

This application is based on and claims priority under 35 U.S.C. §119 to Korean Patent Application No. 10-2023-0132057, filed on October 4, 2023, in the Korean Intellectual Property Office.

### BACKGROUND

### 1. Field

The disclosure relates to an ultrasound probe including a structure combinable with a main backing layer of the ultrasound probe, a method of manufacturing the ultrasound probe using the structure, and the structure.

### 2. Description of the Related Art

An ultrasound diagnostic apparatus is an apparatus that obtains an image of a target part in a body of an object by emitting an ultrasonic signal generated from a transducer of an ultrasound probe from a body surface of the object toward the target part in the body and receiving information about the ultrasonic signal (ultrasound echo signal) reflected from the object.

The ultrasound diagnostic apparatus is widely used in the field of medical diagnosis because it is more stable than an X-ray imaging apparatus because there is no exposure to radiation, etc., may display images in real time, is cheaper than a magnetic resonance imaging apparatus, and is portable.

On the other hand, because the ultrasound probe has a complex structure to drive the transducer, a lot of manpower and time are wasted during manufacturing process thereof.

US 2016/066885 A1 relates to a probe, and more particularly to a probe for an ultrasonic imaging apparatus so as to generate images of the inside of a target object using ultrasonic waves, and a method for manufacturing the same.

US 2018/062069 A1 provides an ultrasound probe and a method of manufacturing the ultrasound probe via which a thin piezoelectric layer and a thin matching layer are formed. The method includes: preparing a backing layer having first and second surfaces with different heights due to forming a groove in the backing layer, wherein first and second electrodes are exposed on the first and second surfaces, respectively; forming a third electrode that is in contact with the first electrode; forming a base piezoelectric unit on the third electrode, the base piezoelectric unit including a piezoelectric layer; forming a piezoelectric unit by removing an upper region of the base piezoelectric unit; and forming a fourth electrode on the backing layer and the piezoelectric unit

EP 2 025 414 A1 relates to a diagnostic ultrasound transducer that has an array of electro-acoustic elements, such as PZT elements, mounted on a backing block. At least one matching layer is mounted on the array, as well as (for most implementations) a lens.

### SUMMARY

It is an aspect of the disclosure to provide a structure capable of facilitating the manufacture of an ultrasound probe, a method of manufacturing an ultrasound probe using the structure, and an ultrasound probe including the structure.

Additional aspects of the disclosure will be set forth in part in the description which follows and, in part, will be obvious from the description, or may be learned by practice of the disclosure.

The invention is defined by claim 1. Regarding the method of manufacturing an ultrasound probe the invention is defined by claim 15.

In accordance with an aspect of the disclosure, an ultrasound probe includes a main backing layer, a piezoelectric layer provided to extend in a horizontal direction on an upper side of the main backing layer to be electrically connected to a first electrode and a second electrode, an acoustic matching layer provided to extend in the horizontal direction on an upper side of the piezoelectric layer, a sub-backing layer provided to extend in a vertical direction below a lower side of the acoustic matching layer, and a flexible printed circuit board including a signal electrode in contact with the first electrode and a ground electrode, wherein the sub-backing layer includes a first backing layer, a first conductive layer, a second backing layer, and a second conductive layer which are stacked in the horizontal direction, the first conductive layer is in contact with the second electrode to be electrically connected to the second electrode, the second backing layer includes a first via hole for electrically connecting the first conductive layer and the second conductive layer, and the second conductive layer is electrically connected to the ground electrode.

The sub-backing layer may include a third backing layer, a third conductive layer, a fourth backing layer, and a fourth conductive layer which are stacked in the horizontal direction, the first backing layer, the first conductive layer, the second backing layer, and the second conductive layer may be provided on a first side of the main backing layer, the third backing layer, the third conductive layer, the fourth backing layer, and the fourth conductive layer may be provided on a second side of the main backing layer opposite to the first side, the third conductive layer may be in contact with the second electrode to be electrically connected to the second electrode, the fourth backing layer may include a second via hole for electrically connecting the third conductive layer and the fourth conductive layer, and the fourth conductive layer may be electrically connected to the ground electrode.

The ultrasound probe may further include a conductive adhesive material adhering the second conductive layer and the ground electrode.

The flexible printed circuit board and the sub-backing layer may be manufactured integrally.

The sub-backing layer may further include an insulator provided to electrically block the second conductive layer and the piezoelectric layer.

The sub-backing layer may further include an alignment hole penetrating the first backing layer, the first conductive layer, the second backing layer, and the second conductive layer.

The first backing layer and the second backing layer may be polytetrafluoroethylene (PTFE) composites.

The main backing layer may be a polytetrafluoroethylene (PTFE) composite, and the main backing layer, the flexible printed circuit board, and the sub-backing layer may be manufactured integrally.

The first conductive layer may be a metal layer, and the second conductive layer may be a layer formed by an adhesive material adhering the second backing layer and the ground electrode.

The first backing layer and the second backing layer may be made of a material having a sound absorption performance of 40 dB/cm/5 MHz or more.

The first electrode is provided on a lower surface of the piezoelectric layer, and the second electrode may be provided on a lower surface of the acoustic matching layer.

The first electrode may be provided on one region of a lower surface of the piezoelectric layer, and the second electrode may be provided on a lower surface of the acoustic matching layer, a side surface of the piezoelectric layer, and the other region of the lower surface of the piezoelectric layer.

The ultrasound probe may further include a printed circuit board provided to output a driving signal for driving the piezoelectric layer, wherein the flexible printed circuit board further may include a connector electrically connected to the printed circuit board, and the ground electrode and the signal electrode may be electrically connected to the printed circuit board through the connector.

The driving signal may be a signal for applying a voltage between the first electrode and the second electrode.

In accordance with another aspect of the disclosure, a method of manufacturing an ultrasound probe includes adhering a structure including a flexible printed circuit board including a signal electrode and a ground electrode and a sub-backing layer to a main backing layer, folding the structure such that the sub-backing layer is disposed on opposite sides of the main backing layer, stacking a piezoelectric layer on the structure, and stacking an acoustic matching layer on the piezoelectric layer, wherein the sub-backing layer includes a first backing layer, a first conductive layer, a second backing layer, and a second conductive layer which are stacked in a width direction of the main backing layer by the folding of the structure, the first conductive layer is in contact with a lower surface of the acoustic matching layer by the stacking of the acoustic matching layer, the second backing layer includes a via hole for electrically connecting the first conductive layer and the second conductive layer, and the second conductive layer is electrically connected to the ground electrode through the via hole.

The signal electrode may be in contact with a lower surface of the piezoelectric layer by the stacking of the piezoelectric layer.

The structure may include a groove formed by the sub-backing layer, and the stacking of the piezoelectric layer on the structure may include inserting the piezoelectric layer into the groove.

The sub-backing layer may include a plurality of alignment holes penetrating the first backing layer, the first conductive layer, the second backing layer, and the second conductive layer, and the folding of the structure may include aligning the structure such that the plurality of alignment holes is symmetrical with respect to the main backing layer.

The method of manufacturing the ultrasound probe may further include electrically connecting the flexible printed circuit board with a printed circuit board provided to output a driving signal for driving the piezoelectric layer.

In accordance with another aspect of the disclosure, a structure includes a flexible printed circuit board including a signal electrode and a ground electrode and a sub-backing layer and capable of being combined with a main backing layer of an ultrasound probe, wherein the sub-backing layer includes a first backing layer, a first conductive layer, a second backing layer, and a second conductive layer which are stacked on the flexible printed circuit board, the second backing layer includes a via hole for electrically connecting the first conductive layer and the second conductive layer, and the second conductive layer is electrically connected to the ground electrode through the via hole.

### BRIEF DESCRIPTION OF THE DRAWINGS

These and/or other aspects of the disclosure will become apparent and more readily appreciated from the following description of the embodiments, taken in conjunction with the accompanying drawings of which:
FIG. 1 illustrates an outer appearance of an ultrasound diagnostic apparatus according to an example not forming part of the present invention;
FIG. 2 is a control block diagram of the ultrasound diagnostic apparatus according to an embodiment of the disclosure;
FIG. 3 is a control block diagram of an ultrasound probe according to an embodiment of the disclosure;
FIG. 4 is a view for explaining a structure for manufacturing the ultrasound probe according to an embodiment of the disclosure;
FIG. 5 illustrates an example of a layered structure of the structure according to an embodiment of the disclosure;
FIG. 6 illustrates an example of a process in which a main backing layer, a piezoelectric layer, and an acoustic matching layer are combined into the structure according to an embodiment of the disclosure;
FIG. 7 illustrates an example of a cross-sectional view of the main backing layer, the piezoelectric layer, and the acoustic matching layer combined into the structure according to an embodiment of the disclosure;
FIGS. 8A, 8B, and 8C are views for explaining alignment holes formed in the structure according to an embodiment of the disclosure;
FIGS. 9A and 9B are views for explaining materials of the main backing layer and a sub-backing layer of the ultrasound probe according to an embodiment of the disclosure;
FIG. 10 is a view illustrating an example in which a conductive layer of the sub-backing layer of the ultrasound probe according to an embodiment of the disclosure is formed by an adhesive material;
FIG. 11 illustrates an example of electrodes formed around the piezoelectric layer of the ultrasound probe according to an embodiment of the disclosure; and
FIG. 12 is a flowchart illustrating an example of a method of manufacturing the ultrasound probe according to an embodiment of the disclosure.

### DETAILED DESCRIPTION

Configurations shown in the embodiments and the drawings described in the specification are only the preferred embodiments of the disclosure, and thus it is to be understood that various modified examples, which may replace the embodiments and the drawings described in the specification, are possible when filing the application.

The terms used herein are for the purpose of describing the embodiments and are not intended to restrict and/or to limit the disclosure.

For example, the singular expressions herein may include plural expressions, unless the context clearly dictates otherwise.

The terms "comprises" and "has" are intended to indicate that there are features, numbers, steps, operations, components, parts, or combinations thereof described in the specification, and do not exclude the presence or addition of one or more other features, numbers, steps, operations, components, parts, or combinations thereof.

The terms including ordinal numbers, such as "first," "second," etc., are used to distinguish one component from another component and do not limit the one component.

In this specification, a first probe and a second probe refer to different configurations having different bodies.

In addition, terms such as "~unit," "~part," "-block," "~member," "~module," etc., may denote a unit for processing at least one function or operation. For example, the terms may refer to at least one hardware such as a field-programmable gate array (FPGA)/an application specific integrated circuit (ASIC), at least one software stored in a memory, or at least one process processed by a processor.

In this specification, "probe" refers to an "ultrasound probe" capable of transmitting and receiving ultrasonic signals.

In this specification, "image" may refer to multi-dimensional data composed of discrete image elements. For example, the image may include, but is not limited to, a medical image (ultrasound image, CT image, MR image) of an object obtained by an ultrasound device, a CT device, or an MRI device.

In this specification, an object may include a person, an animal, or a part of a person or an animal. For example, the object may include organs such as the liver, heart, uterus, brain, breast, and abdomen, or blood vessels. In addition, the object may include a phantom, and the phantom may refer to a material having a volume very close to the density and effective atomic number of a living organism. For example, the phantom may be a spherical phantom with characteristics similar to the human body.

In this specification, an ultrasound image may refer to an image obtained by emitting an ultrasonic signal generated from a transducer of a probe to an object and receiving information about an echo signal reflected from the object. In addition, ultrasound image may be implemented in various ways. For example, the ultrasound image may be at least one of an A-mode (amplitude mode) image, a B-mode (brightness mode) image, a C-mode (color mode) image, and a D-mode (Doppler mode) image. According to an embodiment of the disclosure, the ultrasound image may be a 2-dimensional image or a 3-dimensional image.

In this specification, a transducer of a probe may include a piezoelectric layer.

In addition, throughout the specification, an "operator" may be a doctor, nurse, clinical pathologist, medical imaging expert, etc., as a medical professional, and may be a technician who repairs a medical device, but is not limited thereto.

Hereinafter, an embodiment of the disclosure will be described in detail with reference to the accompanying drawings. The same reference numbers or symbols shown in the accompanying drawings may indicate parts or components that perform substantially the same functions.

Hereinafter, the operating principle and embodiments of the disclosure will be described with reference to the accompanying drawings.

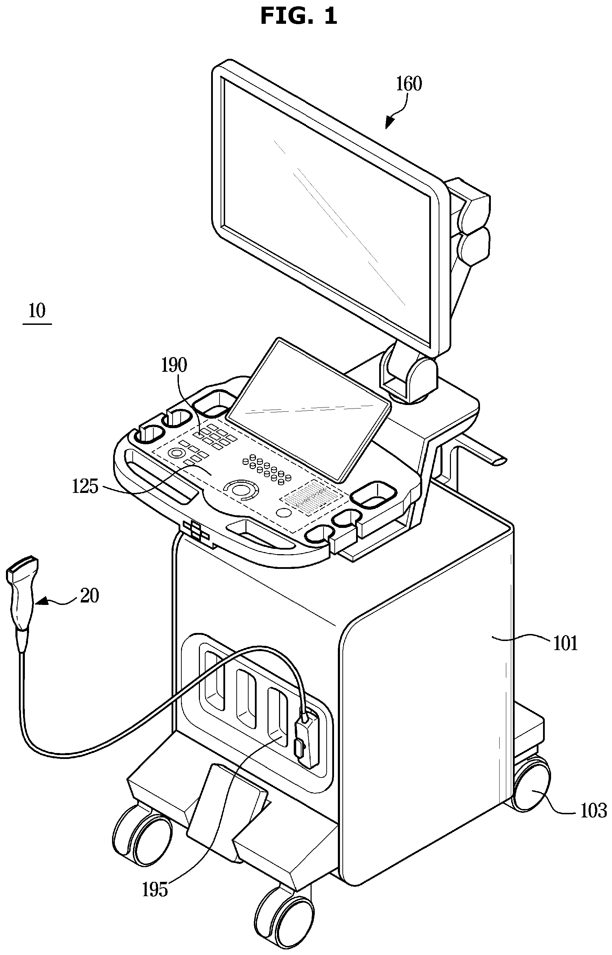

FIG. 1 illustrates an outer appearance of an ultrasound diagnostic apparatus according to an embodiment of the disclosure.

Referring to FIG. 1, the ultrasound diagnostic apparatus 10 according to an example not forming part of the present inventionmay include a main body 101 in which various components are embedded, a plurality of casters 103 provided to move the main body 101, a control panel 105 provided in the main body 101, and a display 160 provided to display ultrasound images.

The plurality of casters 103 may be provided at a lower portion of the main body 101 to move the ultrasound diagnostic apparatus 10. A user may fix or move the ultrasound diagnostic apparatus 10 using the plurality of casters 103. The ultrasound diagnostic apparatus 10 described as above is called a cart-type ultrasound diagnostic apparatus.

However, the ultrasound diagnostic apparatus 10 is not limited to the cart-type ultrasound diagnostic apparatus, and may also be implemented as a portable-type ultrasound diagnostic apparatus. Examples of portable ultrasound diagnostic devices include, but are not limited to, a fax viewer, smart phone, laptop computer, PDA, tablet PC, etc.

The plurality of casters 103 may include electronic casters which are operated by electrical signals. The electronic caster may provide a driving force in a direction in which the user moves the main body 101, and may also be fixed depending on the intention of the user.

The control panel 105 may be provided on the front of the main body 101. An input device 190 provided to receive user input may be formed on the control panel 105, and the user may input commands for selecting a probe, starting diagnosis, selecting a diagnosis area, selecting a diagnosis type, and selecting a mode for ultrasound images through the input device 190.

The display 160 may be provided at an upper portion of the main body 101. The display 160 may be implemented as at least one of various display panels, such as a liquid crystal display (LCD) panel, a light emitting diode (LED) panel, and an organic light emitting diode (OLED) panel.

In a case in which the display 160 is configured as a touch screen, the display 160 may also be used as the input device 190. The touch screen may be configured to detect not only a touch input location and a touched area, but also a touch pressure. The touch screen may also be configured to detect proximity touch as well as real-touch.

The display 160 may include at least one display. In a case in which the display 160 is composed of a plurality of displays, the plurality of displays may display different images simultaneously. For example, one display may display a 2D ultrasound image and another display may display a 3D ultrasound image. Or, one display may display a B-mode image and the other display may display a contrast agent image. Or, one display may display an ultrasound image and the other display may display waveform information of a transmitted signal.

According to one embodiment, the display 160 may output an ultrasound image obtained by the ultrasound probe 20.

An operator such as a doctor may diagnose a specific disease using the ultrasound image displayed on the display 160, and a region in which the ultrasound image is obtained may vary depending on a disease to be diagnosed.

One or more probe holders for mounting the probe 20 may be provided on an outer circumferential surface of the main body 101. Therefore, when the user does not use the probe 20, the user may store the probe 20 by mounting the probe 20 in the probe holder.

At least one slot 195 to which the probe 20 may be connected may be provided in the main body 101.

The at least one slot 195 may be provided in plural numbers, and one probe may be typically connected to each of the plurality of slots 195.

The at least one slot 195 may be a female connector and may be physically coupled with a connector provided at one end of a cable of the probe 20. However, the type of the at least one slot 195 is not limited thereto.

The connector provided at the one end of the cable of the probe 20 may be a male connector. However, the type of the connector of the probe 20 is not limited thereto.

For example, in a case in which the at least one slot 195 is a male connector, the connector provided at the one end of the cable of the probe 20 may be a female connector.

The ultrasound diagnostic apparatus 10 may generate an ultrasound image based on ultrasound data obtained from the probe 20 connected to the at least one slot 195, and display the generated ultrasound image on the display 160.

For example, the operator may select one of a plurality of the probes connected to the plurality of slots 195 using the input device 190 provided on the control panel 105, and the ultrasound diagnostic apparatus 10may generate an ultrasound image based on ultrasound data obtained from the one probe selected depending on user input.

According to various embodiments, the probe 20 may be a wireless probe to transmit ultrasound data (or ultrasound images) to the ultrasound diagnostic apparatus 10 through wireless communication.

For example, each of the probes 20 may be one of a linear probe, a convex probe, a sector probe, an endoscopic probe, a vaginal probe, a transurethral probe, an insertion-type probe capable of being inserted into an object, a bi-plane probe, etc.

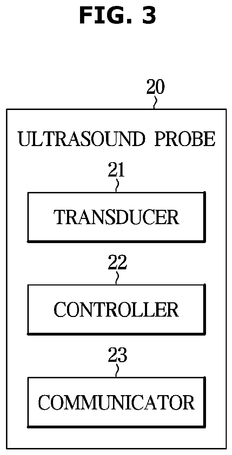

The probe 20 may include a transducer 21 (see FIG. 3) provided to transmit and receive ultrasonic signals to an object.

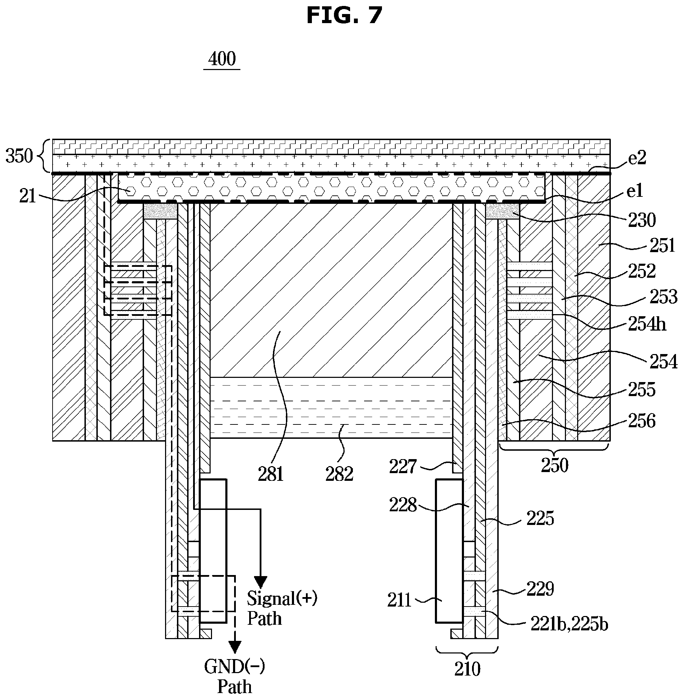

As will be described later, the transducer 21 (FIG. 3) may include the piezoelectric layer 21 (FIG. 7).

The piezoelectric layer may include a plurality of piezoelectric elements. The plurality of piezoelectric elements may be arranged in a one-dimensional array or a two-dimensional array.

The piezoelectric element may convert ultrasonic signals and electrical signals into each other. As an example, the piezoelectric element may be implemented as a piezoelectric ultrasonic transducer using the piezoelectric effect. To this end, the piezoelectric element may include a piezoelectric material or a piezoelectric thin film. When an alternating current is applied to the piezoelectric material or piezoelectric thin film from an internal power storage device such as a battery or an external power supply, the piezoelectric material or piezoelectric thin film vibrates at a predetermined frequency, and ultrasound of a predetermined frequency is generated depending on the vibration frequency.

Conversely, when echo ultrasound of a predetermined frequency reaches the piezoelectric material or piezoelectric thin film, the piezoelectric material or piezoelectric thin film vibrates depending on a frequency of the arrived echo ultrasound, and the piezoelectric material or piezoelectric thin film outputs an alternating current with a frequency corresponding to the vibration frequency.

Also, the piezoelectric element may be implemented by another piezoelectric element, such as a magnetostrictive ultrasonic transducer element using the magnetostrictive effect of a magnetic material or a capacitive micromachined ultrasonic transducer element (cMUT element) that transmits and receives ultrasonic waves using the vibration of hundreds or thousands of micromachined thin films.

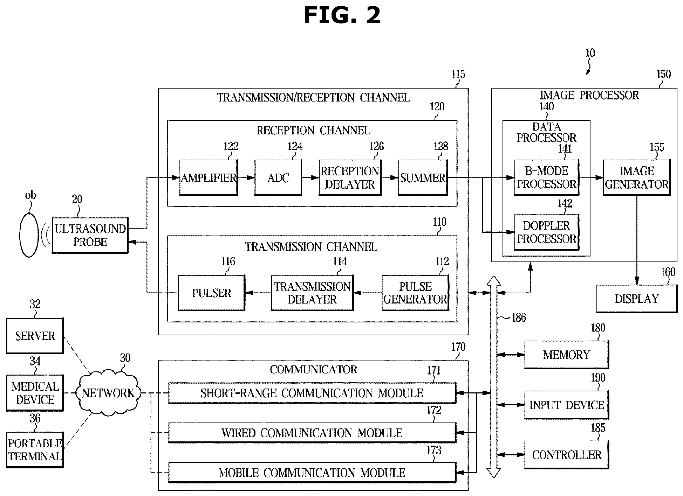

FIG. 2 is a control block diagram of the ultrasound diagnostic apparatus according to an example not forming part of the present invention.

Referring to FIG. 2, the ultrasound diagnostic apparatus 10 according to an example not forming part of the present inventionmay include an ultrasound transmission/reception channel 115, an image processor 150, a communicator 170, the display 160, a memory 180, the input device 190, and a controller 185, and the above components may be connected to each other via a bus 186.

The ultrasound transmission/reception channel 115 may include a plurality of transmission channels and a plurality of reception channels. Hereinafter, for convenience of explanation, the plurality of transmission channels or the plurality of reception channels are collectively referred to as a plurality of channels.

The probe 20 may transmit an ultrasonic signal (transmission signal) to an object ob in response to a driving signal applied from the ultrasound transmission/reception channel 115 and may receive an ultrasonic signal (echo signal) reflected from the object ob.

The probe 20 may include a transducer, and the transducer vibrates in response to an electrical signal transmitted from the ultrasound diagnostic apparatus 10 and generates ultrasonic waves, which is acoustic energy.

In a case in which the probe 20 is a wired probe connected to the ultrasound diagnostic apparatus 10 by wire, the transducer may vibrate in response to an electrical signal transmitted from the ultrasound diagnostic apparatus 10 by wire and generate ultrasonic waves, which is acoustic energy.

In a case in which the probe 20 is a wireless probe that performs wireless communication with the ultrasound diagnostic apparatus 10, the transducer vibrates in response to an electrical signal transmitted from the ultrasound diagnostic apparatus 10 through wireless communication and generates ultrasonic waves, which is acoustic energy.

In the case in which the probe 20 is a wireless probe, some components of the ultrasound diagnostic apparatus 10 (e.g., the transmission/reception channel 115, and/or image processor 150, and/or display 160) may be included in the probe 20. For example, in the case in which the probe 20 is a wireless probe, the transmission/reception channel 115 may be embedded in the probe 20. As another example, in a case in which the probe 20 is a wireless probe, the transmission/reception channel 115 and the image processor 130 may be embedded in the probe 20.

The transmission channel 110 may include a plurality of transmission channels to supply driving signals to the probe 20, and may include a pulse generator 112, a transmission delayer 114, and a pulser 116. The pulse generator 112 may generate pulses for forming transmission ultrasonic waves depending on a predetermined pulse repetition frequency (PRF), the transmission delayer 114 may apply a delay time for determining transmission directionality to the pulses. The pulses to which the delay time is applied may respectively correspond to the plurality of piezoelectric elements included in the piezoelectric layer 21 of the probe 20.

That is, the transmission channel 110 may be provided with transmission channels in the plurality of piezoelectric elements included in the piezoelectric layer 21 of the probe 20 and transmit a transmission signal to each of the piezoelectric elements through each of the plurality of transmission channels.

According to various embodiments, each of the plurality of transmission channels may correspond to at least one of the piezoelectric elements. For example, any one of the plurality of transmission channels may transmit a transmission signal to one or more of the piezoelectric elements.

The pulser 116 may apply the driving signal (or driving pulse) to the probe 20 with timing corresponding to each pulse to which the delay time is applied.

The reception channel 120 may include a plurality of reception channels for generating ultrasound data by processing ultrasonic signals (echo signals) received from the probe 20 and may include an amplifier 122, an analog digital converter (ADC) 124, a reception delayer 126, and a summer 128. The amplifier 122 amplifies the echo signal for each receiving channel, and the ADC 124 converts the amplified echo signal into analog to digital. The reception delayer 126 applies the delay time for determining the reception directionality to the digitally converted echo signal, and the summer 128 generates ultrasound data by summing the echo signals processed by the reception delayer 126. On the other hand, the reception channel 120 may not include the amplifier 122 depending on an implementation type thereof. That is, in a case in which the sensitivity of the probe 20 is improved or the number of processing bits of the ADC 124 increases, the amplifier 122 may be omitted.

The image processor 150 may generate an ultrasound image through a scan conversion process on the ultrasound data generated by the ultrasound transmission/reception channel 115.

The ultrasound image may be not only a gray scale ultrasound image obtained by scanning the object ob in A-mode (amplitude mode), B-mode (brightness mode), and M-mode (motion mode), but also may be a Doppler image expressing the moving object ob using the Doppler effect. The Doppler image may include a blood flow Doppler image (also called a color flow image) showing blood flow, a tissue Doppler image showing a tissue movement, and a spectral Doppler image showing a movement speed of the object ob as a waveform.

The data processor 140 may process ultrasound data.

A B-mode processor 141 extracts and processes a B-mode component from the ultrasound data. An image generator 155 may generate an ultrasound image in which the intensity of a signal is expressed as brightness based on the B-mode component extracted by the B-mode processor 141.

Likewise, a Doppler processor 142 may extract a Doppler component from the ultrasound data and the image generator 155 may generate a Doppler image (for example, a color flow image, etc.) expressing the movement of the object ob in color or waveform based on the extracted Doppler component.

The image generator 155 may generate a 3D ultrasound image through a volume rendering process for volume data and may also generate an elastic image that images a degree of deformation of the object ob depending on a pressure.

In addition, the image generator 155 may express a variety of additional information in text and graphics on the ultrasound image. The generated ultrasound image may be stored in the memory 180.

The image generator 155 may generate an ultrasound image based on processing of the ultrasonic signal received through the probe 20.

The ultrasound image generated through the image generator 155 may be outputted on the display 160.

The communicator 170 is connected to a network 30 by wire or wirelessly to communicate with an external device or server. The communicator 170 may exchange data with a server of a hospital connected through a picture archiving and communication system (PACS) or with another medical device within the hospital. The communicator 170 may also perform data communication according to the digital imaging and communications in medicine (DICOM) standard.

The communicator 170 may transmit and receive data related to the diagnosis of the object ob, such as ultrasound images, ultrasound data, and Doppler data of the object ob, through the network 30 and may also transmit and receive medical images captured by another medical device, such as CT, MRI, and X-ray. In addition, the communicator 170 may receive information about diagnosis history or treatment schedule of a patient from the server and use the received information to diagnose the object ob. Furthermore, the communicator 170 may perform data communication not only with servers or medical devices within the hospital, but also with portable terminals of doctors or patients.

The communicator 170 may also transmit ultrasound images to external devices through the network 30.

The communicator 170 may exchange data with a server 32, a medical device 34, or a portable terminal 36 by being connected to the network 30 by wire or wirelessly. The communicator 170 may include one or more components that enable communication with an external device, and may include, for example, a short-range communication module 171, a wired communication module 172, and a mobile communication module 173.

The short-range communication module 171 refers to a module for short-distance communication within a predetermined distance. Short-range communication technology according to an embodiment of the disclosure may include wireless LAN, Wi-Fi, Bluetooth, ZigBee, Wi-Fi Direct (WFD), ultra-wideband (UWB), infrared data association (IrDA), Bluetooth Low Energy (BLE), Near Field Communication (NFC), etc., but is not limited thereto.

The wired communication module 172 refers to a module for communication using electrical signals or optical signals, and wired communication technology according to an embodiment of the disclosure may include a pair cable, a coaxial cable, an optical fiber cable, an Ethernet cable, etc.

The mobile communication module 173 transmits and receives wireless signals with at least one of a base station, an external terminal, and a server on a mobile communication network. Herein, the wireless signals may include various types of data according to the transmission/reception of voice call signal, video call signal, or text/multimedia message.

In the case in which the probe 20 is a wireless probe, the probe 20 and the ultrasound diagnostic apparatus may transmit and receive various signals through the short-range communication module 171.

The memory 180 stores a variety of information processed by the ultrasound diagnostic apparatus 10. For example, the memory 180 may store medical data related to the diagnosis of the object ob, such as inputted and outputted ultrasound data and ultrasound images and may also store an algorithm or program to be performed within the ultrasound diagnostic apparatus 10.

The memory 180 may be implemented as various types of storage media, such as a flash memory, hard disk, and EEPROM. In addition, the ultrasound diagnostic apparatus 10 may operate a web storage or cloud server performing a memory storage function on a web.

The input device 190 refers to a means through which a user receives data to control the ultrasound diagnostic apparatus 10. The input device 190 may include hardware components such as a keypad, mouse, touchpad, trackball, and jog switch, but is not limited thereto. The input device 190 may also include a fingerprint recognition sensor to recognize a fingerprint of the user. In addition, the input device 190 may further include various components such as an electrocardiogram measurement module, a respiration measurement module, a voice recognition sensor, a gesture recognition sensor, an iris recognition sensor, a depth sensor, and a distance sensor. In particular, the input device 190 may include a touch screen in which a touch pad forms a layer structure with the display 160 described above.

In this case, the ultrasound diagnostic apparatus 10 may display an ultrasound image in a predetermined mode and a control panel for the ultrasound image on the touch screen. In addition, the ultrasound diagnostic apparatus 10 may detect a touch gesture of the user for the ultrasound image through the touch screen.

The ultrasound diagnostic apparatus 10 may physically include some buttons frequently used by the user among buttons included in a control panel of a general ultrasound device, and the remaining buttons may be provided through the touch screen in the form of a graphical user interface (GUI).

The controller 185 overall controls operations of the ultrasound diagnostic apparatus 10. That is, the controller 185 may control the operations between the probe 20, the ultrasound transmission/reception channel 115, the image processor 150, the communicator 170, the memory 180, and the input device 190, which are illustrated in FIG. 2.

The controller 185 may include at least one memory in which a program performing operations is stored, which will be described later, and at least one processor executing the stored program. The ultrasound transmission/reception channel 115, the image processor 150, and the controller 185 may use separate memories and processors, or may share a memory and a processor.

Some or all of the probe 20, ultrasound transmission/reception channel 115, image processor 150, display 160, communicator 170, memory 180, input device 190, and controller 185 may operate by software modules, but are not limited thereto, and some of the above-described components may operate by hardware. In addition, at least some of the ultrasound transmission/reception channel 115, image processor 150, and communicator 170 may be included in the controller 185, but the disclosure is not limited to this implementation form.

The probe 20 connected to the slot 195 may transmit probe identification information (e.g., identification number information, probe type information, etc.) to the ultrasound diagnostic apparatus 10 through the cable, and the ultrasound diagnostic apparatus 10 may provide a user interface for selecting a probe on the display 160 based on the probe identification information. Accordingly, the operator may select the probe the operator wishes to use through the user interface, and an image generated based on the ultrasonic signal obtained from the selected probe is outputted on the display 160.

The probe 20 performing wireless communication with the ultrasound diagnostic apparatus 10 may transmit the probe identification information (e.g., identification number information, probe type information, etc.) to the ultrasound diagnostic apparatus 10 through the wireless communication, and the ultrasound diagnostic apparatus 10 may provide the user interface for selecting a probe on the display 160 based on the probe identification information. Accordingly, the operator may select the probe the operator wishes to use through the user interface, and an image generated based on the ultrasonic signal obtained from the selected probe is outputted on the display 160.

FIG. 3 is a control block diagram of an ultrasound probe according to an embodiment of the disclosure.

Referring to FIG. 3, the probe 20 according to an embodiment of the disclosure may include the transducer 21, a controller 22, and a communicator 23.

The transducer 21 may receive an electrical signal to generate an ultrasonic signal and may receive an echo signal reflected from an object to output an electrical signal.

The transducer 21 (FIG. 3) may include the piezoelectric layer 21 (FIG. 7), and the piezoelectric layer may include a plurality of piezoelectric elements.

The communicator 23 may include one or more components that enable communication with an external device, and may include, for example, a short-range communication module, a wired communication module, and a mobile communication module.

In the case in which the probe 20 is a wired probe, the communicator 23 may include a wired communication module.

In the case in which the probe 20 is a wireless probe, the communicator 23 may include a wireless communication module.

The controller 22 may overall control operations of the probe 20. For example, the controller 22 may output a driving signal for driving the transducer 21.

In one embodiment, the controller 22 may output the driving signal for driving the transducer 21 based on a control signal received by wire and/or wirelessly from the ultrasound diagnostic apparatus 10.

The controller 22 may include at least one memory in which a program for controlling the probe 20 is stored and at least one processor executing the stored program.

The controller 22 may be implemented by at least one printed circuit board (e.g., a rigid printed circuit board and a flexible printed circuit board).

The controller 22 and the communicator 23 may use separate memories and processors, or may share a memory and a processor.

For example, the controller 22 and the communicator 23 may be implemented on one printed circuit board or may be implemented on different printed circuit boards.

In order for the controller 22 to control the transducer 21, a structure for transmitting an electrical signal to the transducer 21 is required.

In one embodiment, the probe 20 may include a first printed circuit board including the controller 22, and a second printed circuit board (flexible printed circuit board) for transmitting a driving signal outputted from the first printed circuit board to the transducer 21.

The probe 20 includes an acoustic matching layer to prevent attenuation of an ultrasonic signal outputted from the transducer 21 by matching an acoustic impedance of a medium with the object ob.

The acoustic matching layer may match an acoustic impedance of the object ob with an acoustic impedance of the transducer 21.

The probe 20 further includes a backing layer to absorb ultrasonic signals generated from the transducer 21 and traveling rearward (or laterally).

The backing layer may suppress the loss of ultrasonic signals by blocking or reflecting the ultrasonic signals generated from the transducer 21 and traveling rearward (or laterally).

Because the probe 20 includes various components such as the transducer 21, the acoustic matching layer, and the backing layer, in order to manufacture the probe 20, a structure is required to transmit a driving signal outputted from the controller 22 to the transducer 21.

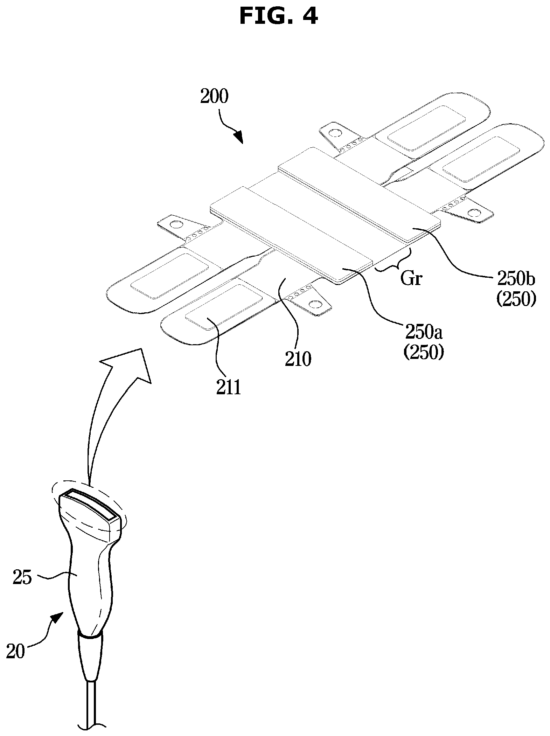

FIG. 4 is a view for explaining a structure for manufacturing the ultrasound probe according to an embodiment of the disclosure.

Referring to FIG. 4, the probe 20 according to an embodiment of the disclosure may include a housing 25 provided to cover various electrical components and includes a structure 200 integrally formed (one-piece).

The structure 200 includes a flexible printed circuit board 210 and a sub-backing layer 250.

A manufacturer of the probe 20 may manufacture the probe 20 by combining a main backing structure 280, the piezoelectric layer 21, and an acoustic matching layer 350 to the structure 200.

The structure 200 includes the flexible printed circuit board 210 and the sub-backing layer 250 stacked on the flexible printed circuit board 210.

The flexible printed circuit board 210 may include a connector 211 capable of being coupled to a printed circuit board (e.g., a rigid printed circuit board including the controller 22) provided inside the probe housing 25.

The connector 211 may be provided on a front or rear surface of the flexible printed circuit board 210.

In one embodiment, the flexible printed circuit board 210 may include the at least one connector 211 (e.g., four connectors), and may receive a signal for driving the transducer 21 from the controller 22 through the at least one connector 211.

The flexible printed circuit board 210 may also transmit an echo signal reflected from the object ob to the controller 22 through the at least one connector 211.

The flexible printed circuit board 210 may be electrically connected to a printed circuit board (e.g., a printed circuit board including the controller 22 and/or the communicator 23) provided to output a driving signal for driving the piezoelectric layer 21 through the connector 211.

The controller 22 may transmit a driving signal corresponding to the plurality of piezoelectric elements included in the transducer 21 to the flexible printed circuit board 210 through the at least one connector 211, and the flexible printed circuit board 210 may transmit the signal transmitted from the controller 22 to the transducer 21.

The at least one the sub-backing layer 250 (e.g., two sub-backing layers 250) may be stacked on the flexible printed circuit board 210. For example, a first sub-backing layer 250a and a second sub-backing layer 250b may be stacked on the flexible printed circuit board 210.

The first sub-backing layer 250a and the second sub-backing layer 250b are spaced apart at a predetermined interval, and a groove Gr is formed by the predetermined interval.

The transducer 21 (piezoelectric layer) may be coupled to the groove Gr.

The structure 200 may be folded based on the groove Gr. When the structure 200 is folded on the basis of the groove Gr, the first sub-backing layer 250a and the second sub-backing layer 250b may be symmetrical to each other with respect to the groove Gr.

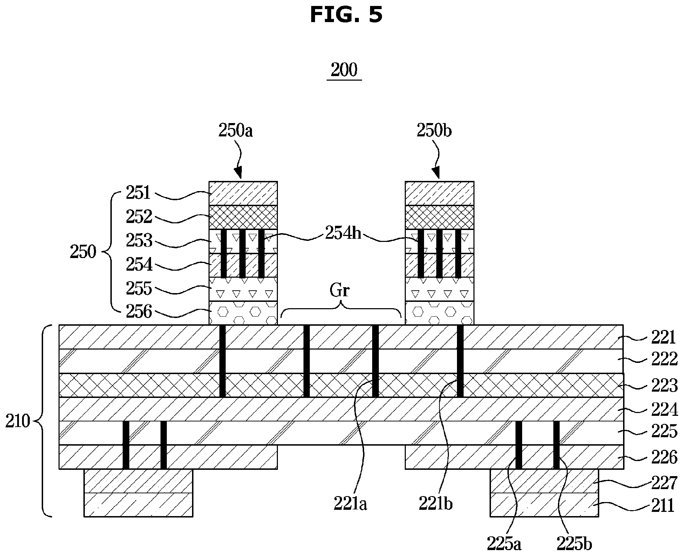

FIG. 5 illustrates an example of a layered structure of the structure according to an embodiment of the disclosure.

Referring to FIG. 5, each of the sub-backing layers 250 (250a and 250b) has a structure in which a first backing layer 251, a first conductive layer 253, a second backing layer 254, and a second conductive layer 255 are stacked.

The first backing layer 251 and the second backing layer 254 may be formed of a material for sound absorption, and the first conductive layer 253 and the second conductive layer 255 may be formed of a conductive material. For example, the first conductive layer 253 may be a metal layer.

That is, the sub-backing layer 250 may include the first backing layer 251, the first conductive layer 253, the second backing layer 254, and the second conductive layer 255, which are stacked in a vertical direction on the flexible printed circuit board 210.

The first backing layer 251 and the first conductive layer 253 may be adhered to each other using an adhesive sheet 252.

In one embodiment, the second conductive layer 255 may be combined to the flexible printed circuit board 210 by an adhesive material 256.

In one embodiment, the adhesive material 256 is a conductive adhesive material.

In the embodiments of the invention, the second backing layer 254 includes at least one via hole 254h to electrically connect the first conductive layer 253 and the second conductive layer 255.

The first conductive layer 253 and the second conductive layer 255 are electrically connected through the at least one via hole 254h formed in the second backing layer 254.

The flexible printed circuit board 210 may have a structure in which polymide (PI) layers and conductive layers (e.g., copper layers) are stacked.

For example, the flexible printed circuit board 210 may have a structure in which conductive layers 221, 224, and 226 are stacked between a plurality of PI layers 222, 225, and 227.

The flexible printed circuit board 210 may include the first signal conductive layer 221, the first PI layer 222 provided below the first signal conductive layer 221, an adhesive sheet 223 provided below the first PI layer 222, the second signal conductive layer 224 provided below the adhesive sheet 223, the second PI layer 225 provided below the second signal conductive layer 224, the third signal conductive layer 226 provided below the second PI layer 225, and the third PI layer 227 provided below the third signal conductive layer 226.

As long as the structure of the flexible printed circuit board 210 is a structure that may satisfy electrical connection relationships between the components of the flexible printed circuit board 210 described above or components of the flexible printed circuit board 210, which will be described later, the structure is not limited to that illustrated in FIG. 5 and may be employed without limitation.

The plurality of conductive layers 221, 224, and 226 may include a signal electrode 228 (see FIG. 7) and a ground electrode 229 (see FIG. 7).

The signal electrode 228 may include at least one of the signal conductive layers 221, 224, and 226.

The signal electrode 228 may be electrically connected to the connector 211 through via holes 221a and 225a formed in the flexible printed circuit board 210.

The ground electrode 229 may include at least one of the signal conductive layers 221, 224, and 226.

The ground electrode 229 may be electrically connected to the connector 211 through via holes 221b and 225b formed in the flexible printed circuit board 210.

The first signal conductive layer 221 may include a portion electrically connected to the groove Gr and a portion electrically connected to the second conductive layer 255 of the sub-backing layer 250.

The portion electrically connected to the groove Gr in the first signal conductive layer 221 and the portion electrically connected to the second conductive layer 255 of the sub-backing layer 250 may be electrically divided from each other.

The portion electrically connected to the groove Gr in the first signal conductive layer 221 may be electrically connected to a portion of the second signal conductive layer 224 through the via hole 221a.

A portion electrically connected to the second conductive layer 255 in the first signal conductive layer 221 may be electrically connected to a portion of the second signal conductive layer 224 through the via hole 221b.

The second signal conductive layer 224 may include a portion electrically connected to the groove Gr and a portion electrically connected to the second conductive layer 255 of the sub-backing layer 250.

A portion electrically connected to the groove Gr in the third signal conductive layer 226 and the portion electrically connected to the second conductive layer 255 of the sub-backing layer 250 may be electrically divided from each other.

The portion electrically connected to the groove Gr may be electrically connected to the piezoelectric layer 21 seated in the groove Gr in a state in which the structure 200 is folded.

The signal electrode 228 and the ground electrode 229 may receive a driving signal from the controller 22 through the connector 211.

The signal electrode 228 and the ground electrode 229 may transmit an electrical signal corresponding to an echo signal to the controller 22 through the connector 211.

According to the structure 200 according to an embodiment of the disclosure, even when the piezoelectric layer 21 is stacked on the groove Gr, the signal electrode 228 and the ground electrode 229 may be electrically connected to the electrodes of the piezoelectric layer 21.

According to the structure 200 according to an embodiment of the disclosure, a process of combining a side backing layer (e.g., the first backing layer 251 and the second backing layer 254), the signal electrode 228, and the ground electrode 229 to the flexible printed circuit board 210 may be omitted.

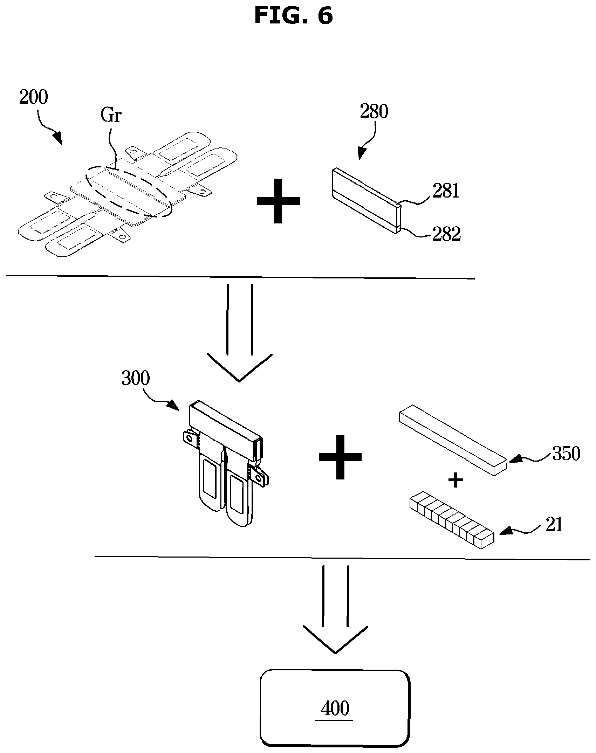

FIG. 6 illustrates an example of a process in which a main backing layer, a piezoelectric layer, and an acoustic matching layer are combined into the structure according to an embodiment of the disclosure.

Referring to FIG. 6, the manufacturer may combine the main backing structure 280 to a lower surface of the flexible printed circuit board 210 corresponding to the groove Gr of the structure 200 according to an embodiment of the disclosure.

In one embodiment, the main backing structure 280 may include a main backing layer 281 and a housing 282.

After combining the main backing structure 280 to the lower surface of the flexible printed circuit board 210 corresponding to the groove Gr of the structure 200 according to an embodiment of the disclosure, the manufacturer may fold the structure 200 such that the flexible printed circuit board 210 surrounds the main backing structure 280.

A head structure 400 may be manufactured when the piezoelectric layer 21 and the acoustic matching layer 350 are stacked on a combined structure 300 manufactured by combining the structure 200 according to an embodiment of the disclosure and the main backing structure 280.

The piezoelectric layer 21 is stacked in the groove Gr of the structure 200, and the acoustic matching layer 350 is stacked above the piezoelectric layer 21.

In one embodiment, the piezoelectric layer 21 may have a width corresponding to the groove Gr of the structure 200, and the acoustic matching layer 350 may have a width corresponding to the groove Gr and sub-backing layer 250 of the structure 200.

Accordingly, in one embodiment, the width of the piezoelectric layer 21 may be shorter than the width of the acoustic matching layer 350.

The head structure 400 may refer to a part forming a head of the probe 20.

When the connector 211 of the flexible printed circuit board 210 is electrically connected to the controller 22 for driving the probe 20 after the head structure 400 is manufactured, the controller 22 may drive the piezoelectric layer 21.

FIG. 7 illustrates an example of a cross-sectional view of the main backing layer, the piezoelectric layer, and the acoustic matching layer combined into the structure according to an embodiment of the disclosure.

Referring to FIG. 7, the head structure 400 according to an embodiment of the disclosure may include the structure 200, the main backing layer 281, the piezoelectric layer 21, and the acoustic matching layer 350.

The main backing layer 281 may be disposed below the piezoelectric layer 21 to absorb or reflect an ultrasonic signal emitted downward from the piezoelectric layer 21.

The housing 282 may be combined to a lower side of the main backing layer 281.

An upper side of the piezoelectric layer 21 may refer to a direction in which ultrasonic signals are emitted from the probe 20.

The piezoelectric layer 21 may be provided to extend in a horizontal direction on an upper side of the main backing layer 281.

The horizontal direction refers to a direction perpendicular to the direction in which ultrasonic signals are emitted (vertical direction).

In one embodiment, the width of the piezoelectric layer 21 may be longer than a width of the main backing layer 281. To this end, in one embodiment, a portion of the second backing layer 254 may be cut to form the groove Gr.

That is, the second backing layer 254 may include a first surface and a second surface having a height different from the first surface.

The acoustic matching layer 350 may be provided to extend in the horizontal direction on the upper side of the piezoelectric layer 21.

Accordingly, the first backing layer 251 and the second backing layer 254 may be provided on a side surface of the piezoelectric layer 21.

The acoustic matching layer 350 may include at least one matching layer (e.g., two matching layers). However, the number of matching layers constituting the acoustic matching layer 350 is not limited thereto.

A first electrode e1 is provided on a lower surface of the piezoelectric layer 21. For example, the lower surface of the piezoelectric layer 21 may be plated with metal. That is, the groove Gr may be plated with metal.

A second electrode e2 is provided on an upper surface of the piezoelectric layer 21 and/or a lower surface of the acoustic matching layer 350. For example, the upper surface of the piezoelectric layer 21 and/or the lower surface of the acoustic matching layer 350 may be plated with metal.

In the processes of manufacturing the probe 20, the manufacturer may manufacture the second electrode e2 by stacking the piezoelectric layer 21 on the structure 200 and then plating metal on the upper surface thereof.

Accordingly, the piezoelectric layer 21 is electrically connected to the first electrode e1 and the second electrode e2.

The piezoelectric layer 21 may be driven based on a potential difference between the first electrode e1 and the second electrode e2.

The first electrode e1, which is a positive electrode, may correspond to an electrode to which a driving voltage for driving the piezoelectric layer is applied.

The second electrode e2, which is a negative electrode, may correspond to a reference electrode of the first electrode e1.

The driving signal for driving the piezoelectric layer 21 may include a signal for applying a voltage between the first electrode e1 and the second electrode e2.

The sub-backing layer 250 is provided to extend in the vertical direction on an upper side of the acoustic matching layer 350.

As the structure 200 is folded based on the groove Gr, the sub-backing layer 250, which is stacked in the vertical direction on the flexible printed circuit board, may be stacked in the horizontal direction on a lower side of the acoustic matching layer 350.

The sub-backing layer 250 may include the first backing layer 251, the first conductive layer 253, the second backing layer 254, and the second conductive layer 255, which are stacked in the horizontal direction.

In the embodiments of the invention, the first conductive layer 253 is in contact with the second electrode e2.

Because metal is plated on the combined structure 300 on which the piezoelectric layer 21 is stacked, the first conductive layer 253 may be electrically connected to the second electrode e2.

In the embodiments of the invention, the second backing layer 254 includes at least one via hole 254h to electrically connect the first conductive layer 253 and the second conductive layer 255.

The at least one via hole 254h electrically connects the first conductive layer 253 and the second conductive layer 255. The second conductive layer 255 is electrically connected to the ground electrode 229 of the flexible printed circuit board 210. As a result, the second electrode e2 may be electrically connected to the ground electrode 229 through the first conductive layer 253, the at least one via hole 254h, and the second conductive layer 255.

In one embodiment, the second conductive layer 255 may be combined to the ground electrode 229 by the conductive adhesive material 256. The conductive adhesive material 256 may include conductive tape, anisotropic conductive film (ACF), and/or anisotropic conductive adhesive (ACA).

The conductive adhesive material 256 may adhere the second conductive layer 255 and the ground electrode 229.

According to this embodiment, the structure 200 may be manufactured integrally by forming the at least one via hole 254h in the second backing layer 254.

The flexible printed circuit board 210 may be electrically connected to a printed circuit board (e.g., the printed circuit board including the controller 22) provided to output the driving signal for driving the piezoelectric layer 21 through the connector 211.

The ground electrode 229 and the signal electrode 228 may be electrically connected to a printed circuit board (e.g., the printed circuit board including the controller 22) through the connector 211.

A ground signal outputted from the connector 211 may be transmitted to the second electrode e2 along the ground electrode 229, the second conductive layer 255, the via hole 254h, and the first conductive layer 253.

A signal outputted from the second electrode e2 may be transmitted to the connector 211 along the first conductive layer 253, the second conductive layer 255, the via hole 254h, and the ground electrode 229.

In one embodiment, the sub-backing layer 250 may include an insulator 230 provided to electrically block the second conductive layer 255 and the first electrode e1. The insulator 230 may be provided between the second conductive layer 255 and the piezoelectric layer 21 in order to electrically block the second conductive layer 255 and the first electrode e1.

When the second conductive layer 255 comes into contact with the piezoelectric layer 21, the second conductive layer 255 is electrically connected to both the first electrode e1 and the second electrode e2 and a short circuit occurs.

In order to prevent the occurrence of the short circuit, the insulator 230 may electrically block the second conductive layer 255 and the first electrode e1.

The insulator 230 may also electrically block the ground electrode 229 and the first electrode e1.

The signal electrode 228 of the flexible printed circuit board 210 is in contact with the first electrode e1 to be electrically connected to the first electrode e1.

The driving signal outputted from the connector 211 may be transmitted to the first electrode e1 through the signal electrode 228.

The signal outputted from the first electrode e1 may be transmitted to the connector 211 through the signal electrode 228.

As described above, the sub-backing layer 250 may include the first sub-backing layer 250a and the second sub-backing layer 250b which are symmetrical with respect to the piezoelectric layer 21 (or the main backing layer 281).

Each of the first sub-backing layer 250a and the second sub-backing layer 250b may include the components described above.

For example, the first sub-backing layer 250a may include the first backing layer 251, the first conductive layer 253, the second backing layer 254, and the second conductive layer 255, and the second sub-backing layer 250b may include a third backing layer corresponding to the first backing layer 251, a third conductive layer corresponding to the first conductive layer 253, a fourth backing layer corresponding to the second backing layer 254, and a fourth conductive layer corresponding to the second conductive layer 255.

The first sub-backing layer 250a is provided on a first side (e.g., left side) of the main backing layer 281, and the second sub-backing layer 250b is provided on a second side (e.g., right side) that is opposite to the first side of the main backing layer 281.

Because descriptions of the third backing layer, third conductive layer, fourth backing layer, and fourth conductive layer overlap with the descriptions of the first backing layer 251, first conductive layer 253, second backing layer 254, and second conductive layer, the descriptions thereof will be omitted.

According to this embodiment, by simply stacking the piezoelectric layer 21 and the matching layer 350 on the structure 200 formed integrally, an electrical relationship between the electrodes for driving the piezoelectric layer 21 and the connector 211 of the flexible printed circuit board 210 may be established.

That is, according to this embodiment, probe manufacturing processes for combining the side backing layer to the main backing layer, combining ground electrodes, and combining signal electrodes may be omitted.

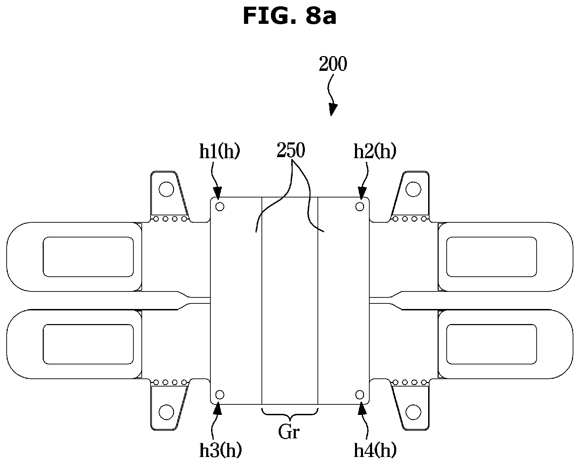

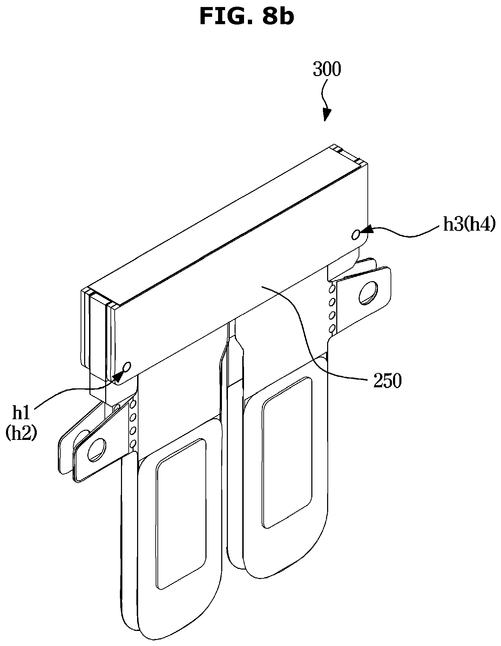

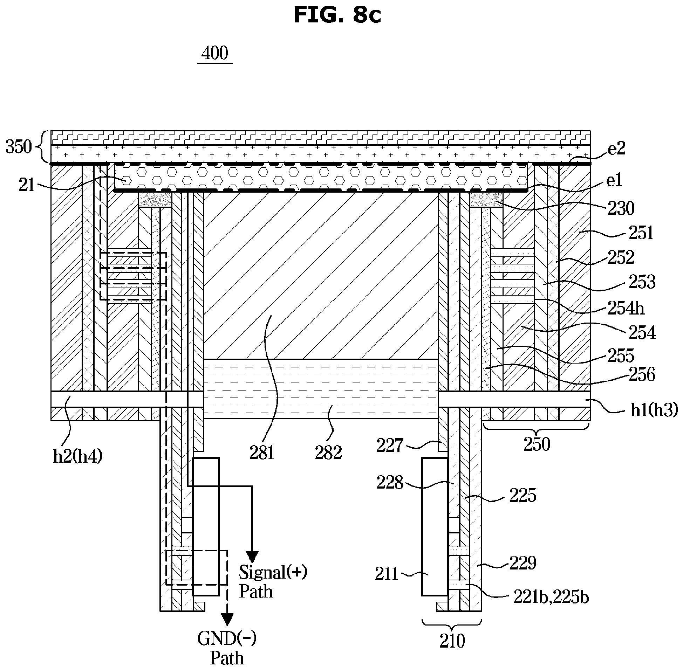

FIGS. 8A, 8B, and 8C are views for explaining alignment holes formed in the structure according to an embodiment of the disclosure.

Referring to FIGS. 8A, 8B, and 8C, the sub-backing layer 250 may include an alignment hole h penetrating the first backing layer 251, the first conductive layer 253, the second backing layer 254, and the second conductive layer 255.

The alignment hole h may include a first hole h1 provided in the first sub-backing layer 250a, and a second hole h2 provided in the second sub-backing layer 250b to be formed at a position symmetrical to the first hole h1 with respect to the groove Gr.

When folding the structure 200, the manufacturer may easily fold the structure 200 so that the structure 200 becomes symmetrical by aligning the positions of the first hole h1 and the second hole h2.

The alignment hole h may include a third hole h3 provided in the first sub-backing layer 250a, and a fourth hole h4 provided in the second sub-backing layer 250b to be formed at a position symmetrical to the third hole h3 with respect to the groove Gr.

The first hole h1 and the third hole h3 may be provided at different positions in the first sub-backing layer 250a.

For example, before the structure 200 is folded, the first hole h1 and the third hole h3 may be provided at an upper left and lower left ends of the first sub-backing layer 250a, respectively.

The second hole h2 and the fourth hole h4 may be provided at different positions in the second sub-backing layer 250b.

For example, before the structure 200 is folded, the second hole h2 and the fourth hole h4 may be provided at an upper right and lower right ends of the second sub-backing layer 250b, respectively.

A distance from a center of the groove Gr to the first hole h1 and a distance from the center of the groove Gr to the second hole h2 may be the same. A distance from the center of the groove Gr to the third hole h3 and a distance from the center of the groove Gr to the fourth hole h4 may be the same.

In one embodiment, the distance from the center of the groove Gr to the first hole h1 and the distance from the center of the groove Gr to the third hole h3 may be the same. The distance from the center of the groove Gr to the second hole h2 and the distance from the center of the groove Gr to the fourth hole h4 may be the same.

When folding the structure 200 to combine the main backing structure 280 to the structure 200, the manufacturer may maintain the symmetry of the structure 200 while observing the alignment hole h.

For example, the housing 282 may be provided with a protrusion provided at a position corresponding to the alignment hole h and having a size corresponding to the alignment hole h.

When folding the structure 200 to combine the main backing structure 280 to the structure 200, the manufacturer may maintain the symmetry of the structure 200 by combining the alignment hole h provided in the structure 200 with the protrusion provided in the housing 282.

According to various embodiments, the sub-backing layer 250 may include an alignment protrusion instead of the alignment hole h. In a case in which the sub-backing layer 250 includes the alignment protrusion instead of the alignment hole h, the housing 282 may include the alignment hole.

According to one embodiment, when the manufacturer folds the structure 200 to combine the main backing structure 280 to the structure 200, the symmetry of the structure 200 may be easily secured.

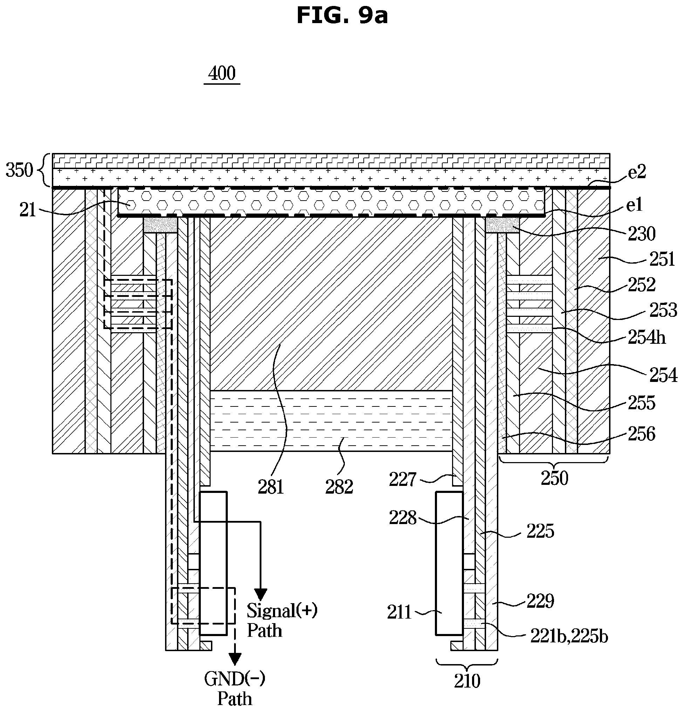

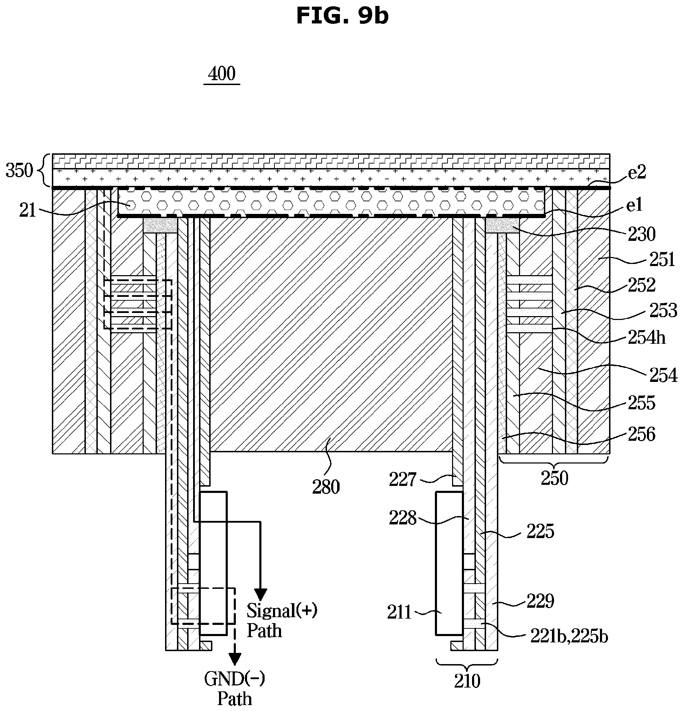

FIGS. 9A and 9B are views for explaining materials of the main backing layer and the sub-backing layer of the ultrasound probe according to an embodiment of the disclosure.

According to various embodiments, a material of the main backing layer 281 and materials of the first backing layer 251 and the second backing layer 254 included in the sub-backing layer 250 may be different from each other or may be the same.

The first backing layer 251 and the second backing layer 254 may be made of a material having a sound absorption performance of 40 dB/cm/5 MHz or more.

Referring to FIG. 9A, the first backing layer 251 and/or the second backing layer 254 included in the sub-backing layer 250 may be a polytetrafluoroethylene (PTFE) composite.

As the second backing layer 254 is formed of the PTFE composite, the via hole 254h may be easily formed in the second backing layer 254.

As the first backing layer 251 is formed of a PTFE composite like the second backing layer 254, uniform sound absorption performance may be secured.

In one embodiment, the main backing layer 281 may also be a PTFE composite like the first backing layer 251 and the second backing layer 254.

In a case in which the main backing layer 281 is formed of the same PTFE composite as the first backing layer 251 and the second backing layer 254, a structure in which the main backing layer 281 is combined to the structure 200 may be manufactured integrally.

In the case in which the main backing layer 281 except the housing 282 is formed of the same PTFE composite as the first backing layer 251 and the second backing layer 254, all components of the combined structure 300 except the housing 282 may be manufactured integrally.

According to this embodiment, a process of combining the main backing layer 281 to the structure 200 may be omitted in the manufacturing processes of the probe 20.

Referring to FIG. 9B, the first backing layer 251, the second backing layer 254, and the main backing structure 280 (the main backing layer 281 and housing 282) may all be the PTFE composites.

That is, the main backing layer 281 and the housing 282 may be implemented as the PTFE composites.

Because the first backing layer 251, the second backing layer 254, the main backing layer 281, and the housing 282 are all the PTFE composites, the combined structure 300 may be manufactured integrally.

According to this embodiment, a process of combing the main backing structure 280 to the structure may be omitted in the probe manufacturing processes.

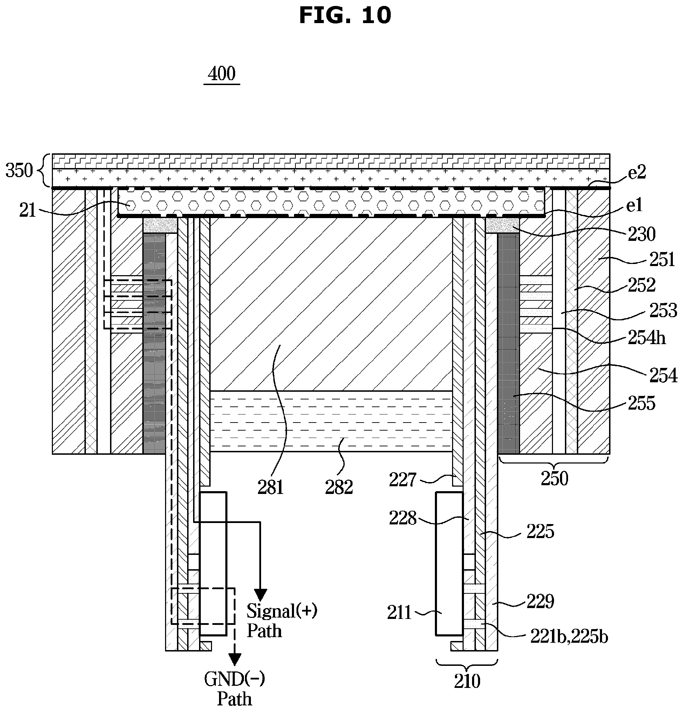

FIG. 10 is a view illustrating an example in which a conductive layer of the sub-backing layer of the ultrasound probe according to an embodiment of the disclosure is formed by an adhesive material.

Referring to FIG. 10, the second conductive layer 255 may be a layer formed by an adhesive material adhering the second backing layer 254 and the ground electrode 229.

That is, the second conductive layer 255 may be an adhesive material rather than a metal layer. The adhesive material may adhere the ground electrode 229 and the second backing layer 254.

In a case in which the second conductive layer 255 is a layer formed by an adhesive material, the conductive adhesive material 256 may be omitted.

In one embodiment, the adhesive material corresponding to the second conductive layer 255 may be a non-conductive adhesive agent. Even in a case in which the ground electrode 229 and the second backing layer 254 are adhere with a non-conductive adhesive agent, in a case in which a thickness of the non-conductive adhesive agent is 3 um or less, the first conductive layer 253 and the ground electrode 229 may be electrically connected through the via hole 254h formed in the second backing layer 254.

According to this embodiment, the second conductive layer 255 may be replaced with an adhesive material layer rather than a metal layer.

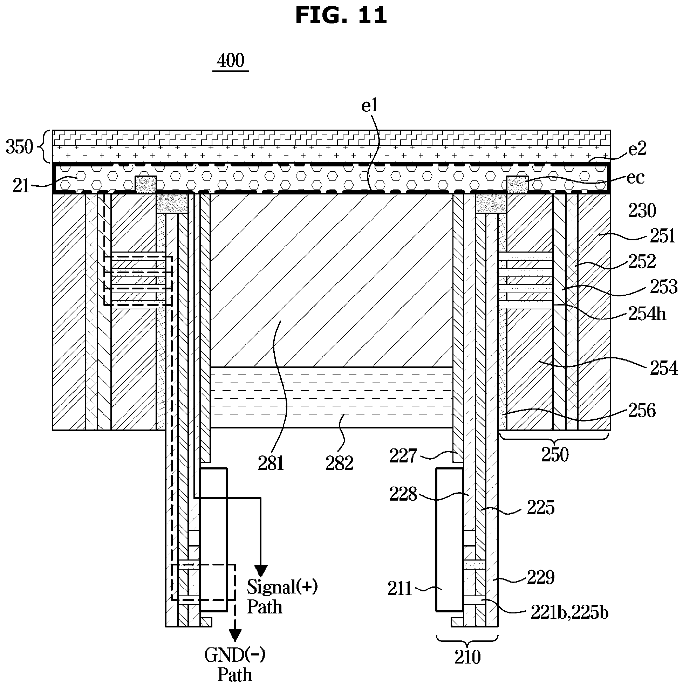

FIG. 11 illustrates an example of electrodes formed around the piezoelectric layer of the ultrasound probe according to an embodiment of the disclosure.

Referring to FIG. 11, the first electrode e1 may be provided on one region of the lower surface of the piezoelectric layer 21, and the second electrode e2 may be provided on the lower surface of the acoustic matching layer 350, the side surface of the piezoelectric layer 21, and the the other region of the lower surface of the piezoelectric layer 21 in which the first electrode e1 is not formed.

According to the embodiment illustrated in FIG. 11, unlike the embodiment illustrated in FIG. 7, the second backing layer 254 may include a first surface and a second surface having the same height as the first surface.

On the other hand, an insulating portion ec may be provided on an upper side of the second backing layer 254. The insulating portion ec may be formed by a process of performing an isolation cut of a portion of the piezoelectric layer 21 in the manufacturing process of the piezoelectric layer 21.

A first region of regions (first region and second region) of the lower surface of the piezoelectric layer 21 separated on the basis of the insulating portion ec may correspond to the first electrode e1, and a second region may correspond to the second electrode e2.

According to this embodiment, by increasing the proportion of an area occupied by the piezoelectric layer 21, the number of the piezoelectric elements included in the piezoelectric layer 21 may be increased compared to the same area.

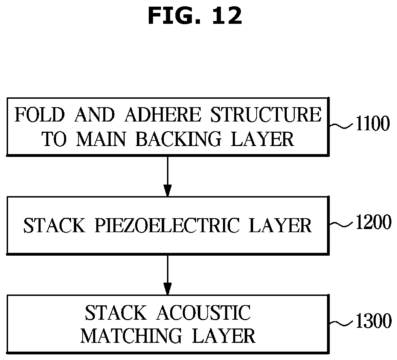

FIG. 12 is a flowchart illustrating an example of a method of manufacturing the ultrasound probe according to an embodiment of the disclosure.

Referring to FIG. 12, a method of manufacturing the ultrasound probe 20 includes folding and adhering the structure 200 to the main backing layer 281 (step 1100).

The folding and adhering of the structure 200 to the main backing layer 281 (step 1100) includes adhering the structure 200 to the main backing layer 281 and folding the structure 200 such that the sub-backing layer 250 is disposed on opposite side surfaces of the main backing layer 281.

In a case in which the sub-backing layer 250 includes a plurality of the alignment holes h penetrating the first backing layer 251, the first conductive layer 253, the second backing layer 254, and the second conductive layer 255, the folding of the structure 200 may include aligning the structure 200 such that the plurality of alignment holes h is symmetrical with respect to the main backing layer 281.

For example, the aligning of the structure 200 such that the plurality of alignment holes h is symmetrical with respect to the main backing layer 281 may include aligning the structure 200 such that the first hole h1 and the second hole h2 are located at the same position in a direction of crossing the main backing layer 281 and/or aligning the structure 200 such that the third hole h3 and the fourth hole h4 are located at the same position in the direction of crossing the main backing layer 281.

The method of manufacturing the ultrasound probe 20 includes stacking the piezoelectric layer 21 on the structure 200 (step 1200).

The stacking the piezoelectric layer 21 on the structure 200 (step 1200) includes inserting the piezoelectric layer 21 into the groove Gr of the structure 200.

The signal electrode 228 included in the structure 200 may be in contact with the lower surface of the piezoelectric layer 21 by the stacking of the piezoelectric layer 21 (step 1200).

The first electrode e1 may be formed in the groove Gr, and thus the signal electrode 228 may drive the piezoelectric layer 21 through the first electrode e1.

The method of manufacturing the ultrasound probe 20 may include stacking the acoustic matching layer 350 on the structure 200 (step 1300).

The second electrode e2 may be formed on the structure 200, and thus the second electrode e2 may be formed on the lower surface of the acoustic matching layer 350.

By the stacking of the piezoelectric layer 21 on the structure 200 (step 1200) and the stacking of the acoustic matching layer 350 on the structure 200 (step 1300), the signal electrode 228 and the ground electrode 229 included in the structure 200 may be naturally electrically connected to the first electrode e1 and the second electrode e2.

Although not shown in the drawing, the method of manufacturing the ultrasound probe 20 may further include electrically connecting the flexible printed circuit board 210 to the printed circuit board provided to output the driving signal for driving the piezoelectric layer 21.

The printed circuit board outputting the driving signal for driving the piezoelectric layer 21 may include the controller 22.

The electrically connecting of the flexible printed circuit board 210 to the printed circuit board outputting the driving signal for driving the piezoelectric layer 21 may include coupling the connector 211 provided on the flexible printed circuit board 210 with a connector of the printed circuit board.

While according to the prior art, all of steps of coupling the signal electrode to the sub-backing layer, coupling the ground electrode to the sub-backing layer, and coupling the sub-backing layer to which the signal electrode and the ground electrode are combined to the main backing layer need to be performed, according to this embodiment, the above steps may be replaced by the step of adhering the structure 200 to the main backing layer 281.

As is apparent from the above, according to an aspect of the disclosure, time and manpower for manufacturing an ultrasound probe can be reduced.

The embodiments disclosed with reference to the accompanying drawings have been described above. It will be understood by those skilled in the art that various changes in form and details may be made therein as defined by the appended claims. The disclosed embodiments are illustrative and should not be construed as limiting.

## Claims

1. An ultrasound probe (20) comprising:
a main backing layer (281);
a piezoelectric layer (21) provided to extend in a first direction, perpendicular to a second direction in which ultrasound signals are emitted by the piezoelectric layer (21), on an upper side of the main backing layer (281);
a first electrode (e1), configured to be provided on, and electrically connected to, a lower side of the piezoelectric layer (21);
a second electrode (e2), configured to be provided on, and electrically connected to, an upper side of the piezoelectric layer (21);
an acoustic matching layer (350) provided to extend in the first direction on an upper side of the piezoelectric layer (21);
a sub-backing layer (250) provided to extend in the second direction below a lower side of the acoustic matching layer (350); and
a flexible printed circuit board (210) comprising a signal electrode (228) in contact with the first electrode (e1) and a ground electrode (229), the flexible printed circuit board (210) extending along the second direction between the sub-backing layer (250) and the main backing layer (281),
wherein the sub-backing layer (250) comprises a first backing layer (251), a first conductive layer (253), a second backing layer (254), and a second conductive layer (255) which are stacked in the first direction,
the first conductive layer (253) is in contact with the second electrode (e2) to be electrically connected to the second electrode,
the second backing layer (254) comprises a first via hole (254h) configured to electrically connect the first conductive layer (253) and the second conductive layer (255), and
the second conductive layer (255) is electrically connected to the ground electrode (229).

2. The ultrasound probe (20) according to claim 1, wherein
the sub-backing layer (250) comprises a third backing layer, a third conductive layer, a fourth backing layer, and a fourth conductive layer which are stacked in the first direction,
the first backing layer (251), the first conductive layer (253), the second backing layer (254), and the second conductive layer (255) are provided on a first side of the main backing layer (281),
the third backing layer, the third conductive layer, the fourth backing layer, and the fourth conductive layer are provided on a second side of the main backing layer (281) opposite to the first side,
the third conductive layer is in contact with the second electrode (e2) to be electrically connected to the second electrode (e2),
the fourth backing layer comprises a second via hole configured to electrically connect the third conductive layer and the fourth conductive layer, and
the fourth conductive layer is electrically connected to the ground electrode (229).

3. The ultrasound probe (20) according to claim 1, further comprising
a conductive adhesive material adhering the second conductive layer (255) and the ground electrode (229).

4. The ultrasound probe (20) according to claim 1, wherein
the flexible printed circuit board (210) and the sub-backing layer (250) are manufactured integrally.

5. The ultrasound probe (20) according to claim 1, wherein
the sub-backing layer (250) further comprises an insulator provided to electrically block the second conductive layer (255) and the piezoelectric layer (21).

6. The ultrasound probe (20) according to claim 1, wherein
the sub-backing layer (250) further comprises an alignment hole penetrating the first backing layer (251), the first conductive layer (253), the second backing layer (254), and the second conductive layer (255).

7. The ultrasound probe (20) according to claim 1, wherein
the first backing layer (251) and the second backing layer (254) are polytetrafluoroethylene (PTFE) composites.

8. The ultrasound probe (20) according to claim 7, wherein
the main backing layer (281) is a polytetrafluoroethylene (PTFE) composite, and
the main backing layer (281), the flexible printed circuit board (210), and the sub-backing layer (250) are manufactured integrally.

9. The ultrasound probe (20) according to claim 1, wherein
the first conductive layer (253) is a metal layer, and
the second conductive layer (255) is a layer formed by an adhesive material adhering the second backing layer (254) and the ground electrode (229).

10. The ultrasound probe (20) according to claim 1, wherein
the first backing layer (251) and the second backing layer (254) are made of a material having a sound absorption performance of 40 dB/cm/5 MHz or more.

11. The ultrasound probe (20) according to claim 1, wherein
the first electrode (e1) is provided on a lower surface of the piezoelectric layer (21), and
the second electrode (e2) is provided on a lower surface of the acoustic matching layer (350).

12. The ultrasound probe (20) according to claim 1, wherein
the first electrode (e1) is provided on one region of a lower surface of the piezoelectric layer (21), and
the second electrode (e2) is provided on a lower surface of the acoustic matching layer (350), a side surface of the piezoelectric layer (21), and the other region of the lower surface of the piezoelectric layer (21).

13. The ultrasound probe (20) according to claim 1, further comprising
a printed circuit board provided to output a driving signal for driving the piezoelectric layer (21),
wherein the flexible printed circuit board (210) further comprises a connector electrically connected to the printed circuit board, and
the ground electrode (229) and the signal electrode (228) are electrically connected to the printed circuit board through the connector.

14. The ultrasound probe (20) according to claim 13, wherein
the driving signal is a signal configured to apply a voltage between the first electrode (e1) and the second electrode (e2).

15. A method of manufacturing an ultrasound probe (20) comprising:
adhering a structure (200) comprising a flexible printed circuit board (210) comprising a signal electrode (228) and a ground electrode (229) and a sub-backing layer (250) to a main backing layer (281), the sub-backing layer (250) comprising two sub-backing layers (250) disposed on an upper side of the flexible printed circuit board (210) and separated by a groove (Gr);
folding the structure (200) such that one of the two sub-backing layers (250) and the other one of the two sub-backing layers (250) are disposed on opposite sides of the main backing layer (281);
stacking a piezoelectric layer (21) on the groove (Gr) of the structure (200); and
stacking an acoustic matching layer (350) on the piezoelectric layer (21),
wherein each of the two sub-backing layers (250) comprises a first backing layer (251), a first conductive layer (253), a second backing layer (254), and a second conductive layer (255) which are stacked by the folding of the structure (200) in a first direction perpendicular to a second direction in which ultrasonic signals are emitted by the piezoelectric layer (21),
first and second electrodes (e1, e2) of the piezoelectric layer (21) are placed on opposite sides of the piezoelectric layer (21),
the signal electrode (228) is in contact with the first electrode (e1),
the first conductive layer (253) is in contact with the second electrode (e2) to be electrically connected to the second electrode (e2),
the second backing layer (254) comprises a via hole configured to electrically connect the first conductive layer (253) and the second conductive layer (255), and
the second conductive layer (255) is electrically connected to the ground electrode (229) through the via hole.

## Patentansprüche

1. Ultraschallsonde (20), umfassend:
eine Hauptträgerschicht (281);
eine piezoelektrische Schicht (21), die so angeordnet ist, dass sie sich in einer ersten Richtung, senkrecht zu einer zweiten Richtung, in der Ultraschallsignale von der piezoelektrischen Schicht (21) ausgesendet werden, auf einer Oberseite der Hauptträgerschicht (281) erstreckt;
eine erste Elektrode (e1), die so ausgebildet ist, dass sie auf einer Unterseite der piezoelektrischen Schicht (21) angeordnet und elektrisch mit dieser verbunden ist;
eine zweite Elektrode (e2), die so ausgebildet ist, dass sie auf einer Oberseite der piezoelektrischen Schicht (21) angeordnet und elektrisch mit dieser verbunden ist;
eine akustische Anpassungsschicht (350), die so angeordnet ist, dass sie sich in der ersten Richtung auf einer Oberseite der piezoelektrischen Schicht (21) erstreckt;
eine Unterträgerschicht (250), die so angeordnet ist, dass sie sich in der zweiten Richtung unterhalb einer Unterseite der akustischen Anpassungsschicht (350) erstreckt; und
eine flexible Leiterplatte (210), die eine mit der ersten Elektrode (e1) in Kontakt stehende Signalelektrode (228) und eine Masseelektrode (229) umfasst, wobei sich die flexible Leiterplatte (210) entlang der zweiten Richtung zwischen der Unterträgerschicht (250) und der Hauptträgerschicht (281) erstreckt,
wobei die Unterträgerschicht (250) eine erste Trägerschicht (251), eine erste leitfähige Schicht (253), eine zweite Trägerschicht (254) und eine zweite leitfähige Schicht (255) umfasst, die in der ersten Richtung übereinander angeordnet sind,
die erste leitfähige Schicht (253) mit der zweiten Elektrode (e2) in Kontakt steht, um elektrisch mit der zweiten Elektrode verbunden zu sein,
die zweite Trägerschicht (254) ein erstes Durchgangsloch (254h) umfasst, das so ausgebildet ist, dass es die erste leitfähige Schicht (253) und die zweite leitfähige Schicht (255) elektrisch verbindet, und
die zweite leitfähige Schicht (255) ist elektrisch mit der Masseelektrode (229) verbunden.

2. Ultraschallsonde (20) nach Anspruch 1, wobei
die Unterträgerschicht (250) eine dritte Trägerschicht, eine dritte leitfähige Schicht, eine vierte Trägerschicht und eine vierte leitfähige Schicht umfasst, die in der ersten Richtung übereinander angeordnet sind,
die erste Trägerschicht (251), die erste leitfähige Schicht (253), die zweite Trägerschicht (254) und die zweite leitfähige Schicht (255) auf einer ersten Seite der Hauptträgerschicht (281) vorgesehen sind,
die dritte Trägerschicht, die dritte leitfähige Schicht, die vierte Trägerschicht und die vierte leitfähige Schicht auf einer der ersten Seite gegenüberliegenden zweiten Seite der Hauptträgerschicht (281) vorgesehen sind,
die dritte leitfähige Schicht mit der zweiten Elektrode (e2) in Kontakt steht, um elektrisch mit der zweiten Elektrode (e2) verbunden zu sein,
die vierte Trägerschicht umfasst ein zweites Durchgangsloch, das so ausgebildet ist, dass es die dritte leitfähige Schicht und die vierte leitfähige Schicht elektrisch verbindet, und
die vierte leitfähige Schicht ist elektrisch mit der Masseelektrode (229) verbunden.

3. Ultraschallsonde (20) nach Anspruch 1, ferner umfassend
ein leitfähiges Klebematerial, das die zweite leitfähige Schicht (255) und die Masseelektrode (229) miteinander verbindet.

4. Ultraschallsonde (20) nach Anspruch 1, wobei
die flexible Leiterplatte (210) und die Unterträgerschicht (250) einstückig hergestellt sind.

5. Ultraschallsonde (20) nach Anspruch 1, wobei
die Unterträgerschicht (250) ferner einen Isolator umfasst, der vorgesehen ist, um die zweite leitfähige Schicht (255) und die piezoelektrische Schicht (21) elektrisch zu trennen.

6. Ultraschallsonde (20) nach Anspruch 1, wobei
die Unterträgerschicht (250) ferner ein Ausrichtungsloch umfasst, das die erste Trägerschicht (251), die erste leitfähige Schicht (253), die zweite Trägerschicht (254) und die zweite leitfähige Schicht (255) durchdringt.

7. Ultraschallsonde (20) nach Anspruch 1, wobei
die erste Trägerschicht (251) und die zweite Trägerschicht (254) Polytetrafluorethylen (PTFE)-Verbundwerkstoffe sind.

8. Ultraschallsonde (20) nach Anspruch 7, wobei
die Hauptträgerschicht (281) ein Polytetrafluorethylen (PTFE)-Verbundwerkstoff ist und
die Hauptträgerschicht (281), die flexible Leiterplatte (210) und die Unterträgerschicht (250) einstückig hergestellt sind.

9. Ultraschallsonde (20) nach Anspruch 1, wobei
die erste leitfähige Schicht (253) eine Metallschicht ist und
die zweite leitfähige Schicht (255) eine Schicht ist, die aus einem Klebematerial gebildet ist, das die zweite Trägerschicht (254) und die Masseelektrode (229) miteinander verbindet.

10. Ultraschallsonde (20) nach Anspruch 1, wobei
die erste Trägerschicht (251) und die zweite Trägerschicht (254) aus einem Material bestehen, das eine Schallabsorptionsleistung von 40 dB/cm/5 MHz oder mehr aufweist.

11. Ultraschallsonde (20) nach Anspruch 1, wobei
die erste Elektrode (e1) auf einer Unterseite der piezoelektrischen Schicht (21) vorgesehen ist und
die zweite Elektrode (e2) auf einer Unterseite der akustischen Anpassungsschicht (350) vorgesehen ist.

12. Ultraschallsonde (20) nach Anspruch 1, wobei
die erste Elektrode (e1) an einem Bereich einer Unterseite der piezoelektrischen Schicht (21) vorgesehen ist und
die zweite Elektrode (e2) an einer Unterseite der akustischen Anpassungsschicht (350), einer Seitenfläche der piezoelektrischen Schicht (21) und dem anderen Bereich der Unterseite der piezoelektrischen Schicht (21) vorgesehen ist.

13. Ultraschallsonde (20) nach Anspruch 1, ferner umfassend
eine Leiterplatte, die zur Ausgabe eines Ansteuersignals zum Ansteuern der piezoelektrischen Schicht (21) vorgesehen ist,
wobei die flexible Leiterplatte (210) ferner einen Steckverbinder umfasst, der elektrisch mit der Leiterplatte verbunden ist, und
die Masseelektrode (229) und die Signalelektrode (228) über den Steckverbinder elektrisch mit der Leiterplatte verbunden sind.

14. Ultraschallsonde (20) nach Anspruch 13, wobei
das Ansteuersignal ein Signal ist, das so ausgelegt ist, dass es eine Spannung zwischen der ersten Elektrode (e1) und der zweiten Elektrode (e2) anlegt.

15. Verfahren zur Herstellung einer Ultraschallsonde (20), umfassend:
Aufbringen einer Struktur (200), die eine flexible Leiterplatte (210) mit einer Signalelektrode (228) und einer Masseelektrode (229) sowie eine Unterträgerschicht (250) umfasst, auf eine Hauptträgerschicht (281), wobei die Unterträgerschicht (250) zwei Unterträgerschichten (250) umfasst, die auf einer Oberseite der flexiblen Leiterplatte (210) angeordnet und durch eine Nut (Gr) voneinander getrennt sind;
Falten der Struktur (200) derart, dass eine der beiden Unterträgerschichten (250) und die andere der beiden Unterträgerschichten (250) auf gegenüberliegenden Seiten der Hauptträgerschicht (281) angeordnet sind;
Aufbringen einer piezoelektrischen Schicht (21) auf die Nut (Gr) der Struktur (200); und
Aufbringen einer akustischen Anpassungsschicht (350) auf die piezoelektrische Schicht (21),
wobei jede der beiden Unterträgerschichten (250) eine erste Trägerschicht (251), eine erste leitfähige Schicht (253), eine zweite Trägerschicht (254) und eine zweite leitfähige Schicht (255) umfasst, die durch das Falten der Struktur (200) in einer ersten Richtung senkrecht zu einer zweiten Richtung, in der Ultraschallsignale von der piezoelektrischen Schicht (21) ausgesendet werden,
eine erste und eine zweite Elektrode (e1, e2) der piezoelektrischen Schicht (21) auf gegenüberliegenden Seiten der piezoelektrischen Schicht (21) angeordnet sind,
die Signalelektrode (228) mit der ersten Elektrode (e1) in Kontakt steht,
die erste leitfähige Schicht (253) mit der zweiten Elektrode (e2) in Kontakt steht, um elektrisch mit der zweiten Elektrode (e2) verbunden zu sein,
die zweite Trägerschicht (254) ein Durchgangsloch umfasst, das so ausgebildet ist, dass es die erste leitfähige Schicht (253) und die zweite leitfähige Schicht (255) elektrisch verbindet, und
die zweite leitfähige Schicht (255) über das Durchgangsloch elektrisch mit der Masseelektrode (229) verbunden ist.

## Revendications

1. Une sonde à ultrasons (20) comprenant :
une couche de support principale (281) ;
une couche piézoélectrique (21) agencée pour s'étendre dans une première direction, perpendiculaire à une seconde direction dans laquelle des signaux ultrasonores sont émis par la couche piézoélectrique (21), sur une face supérieure de la couche de support principale (281) ;
une première électrode (e1), configurée pour être disposée sur la face inférieure de la couche piézoélectrique (21) et pour y être connectée électriquement ;
une deuxième électrode (e2), configurée pour être disposée sur la face supérieure de la couche piézoélectrique (21) et pour y être connectée électriquement ;
une couche d'adaptation acoustique (350) prévue pour s'étendre dans la première direction sur une face supérieure de la couche piézoélectrique (21) ;
une sous-couche de support (250) prévue pour s'étendre dans la deuxième direction sous une face inférieure de la couche d'adaptation acoustique (350); et
une carte de circuit imprimé flexible (210) comprenant une électrode de signal (228) en contact avec la première électrode (e1) et une électrode de masse (229), la carte de circuit imprimé flexible (210) s'étendant dans la deuxième direction entre la sous-couche de support (250) et la couche de support principale (281),
dans laquelle la sous-couche de support (250) comprend une première couche de support (251), une première couche conductrice (253), une deuxième couche de support (254) et une deuxième couche conductrice (255) qui sont empilées dans la première direction,
la première couche conductrice (253) est en contact avec la deuxième électrode (e2) afin d'être connectée électriquement à celle-ci,
la deuxième couche de support (254) comprend un premier trou de passage (254h) configuré pour relier électriquement la première couche conductrice (253) et la deuxième couche conductrice (255), et
la deuxième couche conductrice (255) est reliée électriquement à l'électrode de masse (229).

2. La sonde à ultrasons (20) selon la revendication 1, dans laquelle
la sous-couche de support (250) comprend une troisième couche de support, une troisième couche conductrice, une quatrième couche de support et une quatrième couche conductrice qui sont empilées dans la première direction,
la première couche de support (251), la première couche conductrice (253), la deuxième couche de support (254) et la deuxième couche conductrice (255) sont disposées sur un premier côté de la couche de support principale (281),
la troisième couche de support, la troisième couche conductrice, la quatrième couche de support et la quatrième couche conductrice sont disposées sur un deuxième côté de la couche de support principale (281), opposé au premier côté,
la troisième couche conductrice est en contact avec la deuxième électrode (e2) afin d'être connectée électriquement à celle-ci,
la quatrième couche de support comprend un deuxième trou de passage configuré pour relier électriquement la troisième couche conductrice et la quatrième couche conductrice, et
la quatrième couche conductrice est reliée électriquement à l'électrode de masse (229).

3. La sonde à ultrasons (20) selon la revendication 1, comprenant en outre
un matériau adhésif conducteur faisant adhérer la deuxième couche conductrice (255) et l'électrode de masse (229).

4. La sonde à ultrasons (20) selon la revendication 1, dans laquelle
la carte de circuit imprimé flexible (210) et la sous-couche de support (250) sont fabriquées d'un seul tenant.

5. La sonde à ultrasons (20) selon la revendication 1, dans laquelle
la sous-couche de support (250) comprend en outre un isolant prévu pour isoler électriquement la deuxième couche conductrice (255) et la couche piézoélectrique (21).

6. La sonde à ultrasons (20) selon la revendication 1, dans laquelle
la sous-couche de support (250) comprend en outre un trou d'alignement traversant la première couche de support (251), la première couche conductrice (253), la deuxième couche de support (254) et la deuxième couche conductrice (255).

7. La sonde à ultrasons (20) selon la revendication 1, dans laquelle
la première couche de support (251) et la deuxième couche de support (254) sont des composites de polytétrafluoroéthylène (PTFE).

8. La sonde à ultrasons (20) selon la revendication 7, dans laquelle
la couche de support principale (281) est un composite de polytétrafluoroéthylène (PTFE), et
la couche de support principale (281), la carte de circuit imprimé flexible (210) et la sous-couche de support (250) sont fabriquées d'un seul tenant.

9. La sonde à ultrasons (20) selon la revendication 1, dans laquelle
la première couche conductrice (253) est une couche métallique, et
la deuxième couche conductrice (255) est une couche formée par un matériau adhésif faisant adhérer la deuxième couche de support (254) et l'électrode de masse (229).

10. La sonde à ultrasons (20) selon la revendication 1, dans laquelle
la première couche de support (251) et la deuxième couche de support (254) sont constituées d'un matériau présentant une capacité d'absorption acoustique de 40 dB/cm/5 MHz ou plus.

11. La sonde à ultrasons (20) selon la revendication 1, dans laquelle
la première électrode (e1) est disposée sur une face inférieure de la couche piézoélectrique (21), et
la deuxième électrode (e2) est disposée sur une face inférieure de la couche d'adaptation acoustique (350).

12. La sonde à ultrasons (20) selon la revendication 1, dans laquelle
la première électrode (e1) est disposée sur une région d'une face inférieure de la couche piézoélectrique (21), et
la deuxième électrode (e2) est disposée sur une face inférieure de la couche d'adaptation acoustique (350), sur une face latérale de la couche piézoélectrique (21) et sur l'autre région de la face inférieure de la couche piézoélectrique (21).

13. La sonde à ultrasons (20) selon la revendication 1, comprenant en outre
une carte de circuit imprimé destinée à émettre un signal de commande pour commander la couche piézoélectrique (21),
dans laquelle la carte de circuit imprimé flexible (210) comprend en outre un connecteur relié électriquement à la carte de circuit imprimé, et
l'électrode de masse (229) et l'électrode de signal (228) sont reliées électriquement à la carte de circuit imprimé par l'intermédiaire du connecteur.

14. La sonde à ultrasons (20) selon la revendication 13, dans laquelle
le signal de commande est un signal configuré pour appliquer une tension entre la première électrode (e1) et la deuxième électrode (e2).

15. Procédé de fabrication d'une sonde à ultrasons (20) comprenant :
coller une structure (200) comprenant une carte de circuit imprimé flexible (210) comportant une électrode de signal (228) et une électrode de masse (229), ainsi qu'une sous-couche de support (250), sur une couche de support principale (281), la sous-couche de support (250) comprenant deux sous-couches de support (250) disposées sur une face supérieure de la carte de circuit imprimé flexible (210) et séparées par une rainure (Gr) ;
plier la structure (200) de telle sorte que l'une des deux sous-couches de support (250) et l'autre des deux sous-couches de support (250) soient disposées sur des côtés opposés de la couche de support principale (281) ;
empiler une couche piézoélectrique (21) sur la rainure (Gr) de la structure (200) ; et
empiler une couche d'adaptation acoustique (350) sur la couche piézoélectrique (21),
dans laquelle chacune des deux sous-couches de support (250) comprend une première couche de support (251), une première couche conductrice (253), une deuxième couche de support (254) et une deuxième couche conductrice (255) qui sont empilées par le pliage de la structure (200) dans une première direction perpendiculaire à une deuxième direction dans laquelle des signaux ultrasonores sont émis par la couche piézoélectrique (21),
les première et deuxième électrodes (e1, e2) de la couche piézoélectrique (21) sont placées sur les côtés opposés de la couche piézoélectrique (21), l'électrode de signal (228) est en contact avec la première électrode (e1),
la première couche conductrice (253) est en contact avec la deuxième électrode (e2) afin d'être reliée électriquement à celle-ci,
la deuxième couche de support (254) comprend un trou de passage configuré pour relier électriquement la première couche conductrice (253) et la deuxième couche conductrice (255), et
la deuxième couche conductrice (255) est reliée électriquement à l'électrode de masse (229) par l'intermédiaire du trou de passage.
